**Europäisches Patentamt**

**European Patent Office**

**Office européen des brevets**

(19)

(11) Veröffentlichungsnummer: **0 220 485**
**B1**

(12) **EUROPÄISCHE PATENTSCHRIFT**

(45) Veröffentlichungstag der Patentschrift:
**07.06.89**

(21) Anmeldenummer: **86113023.5**

(22) Anmeldetag: **22.09.86**

(51) Int. Cl.⁴: **C 07 C143/68**, C 08 G 18/38

(54) **Sulfonsäureestergruppen aufweisenden Polyhydroxylverbindungen und ihre Verwendung als Polyolkomponente in Giessharzen.**

(30) Priorität: **01.10.85 DE 3534929**

(43) Veröffentlichungstag der Anmeldung:
**06.05.87 Patentblatt 87/19**

(45) Bekanntmachung des Hinweises auf die Patenterteilung: **07.06.89 Patentblatt 89/23**

(84) Benannte Vertragsstaaten:
**BE DE FR GB IT NL**

(56) Entgegenhaltungen:
**DE–B– 1 128 852**
**GB–A– 1 008 577**

(73) Patentinhaber: **BAYER AG**
**D-5090 Leverkusen 1 Bayerwerk (DE)**

(72) Erfinder: **Prater, Klaus, Dr.**
**Bodelschwinghstrasse 22**
**D-4150 Krefeld (DE)**
Erfinder: **Ürdingen, Walter, Dr.**
**Humperdinckstrasse 41**
**D-5090 Leverkusen (DE)**
Erfinder: **Heine, Heinrich, Dipl.-Ing.**
**Müritzstrasse 36**
**D-5090 Leverkusen (DE)**
Erfinder: **Freitag, Dieter, Dr.**
**Hasenheide 10**
**D-4150 Krefeld (DE)**

## Beschreibung

Die Erfindung betrifft Sulfonsäureestergruppen aufweisende Polyhydroxylverbindungen und ihre Verwendung als Polyolkomponente in Gießharzformulierungen auf Basis von organischen Polyhydroxylverbindungen und organischen Polyisocyanaten.

Die Herstellung von Gießharzen, beispielsweise für den Elektrosektor durch Umsetzung von organischen Polyisocyanaten mit Polyhydroxylverbindungen ist bereits bekannt (vgl. z. B. Kunstoff-Handbuch, Band VII, « Polyurethane » von Becker/Braun Carl Hanser Verlag München, Wien (1983) Seiten 410 bis 425). Derartige Formkörper, die beispielsweise in der Elektroindustrie als Isolatoren Verwendung finden, müssen grundsätzlich blasen- und schaumfrei sein, weswegen gemäß Stand der Technik auch auf eine sorgfältige Entgasung und Trocknung der Ausgangsmaterialien, insbesondere der Polyhydroxylverbindungen, Wert gelegt wird. Im allgemeinen werden den Reaktionsgemischen zusätzlich noch Wasserabsorptionsmittel wie beispielsweise (wasserfreie) Zeolithe zugesetzt, um eine Blasenbildung, hervorgerufen durch die unter Abspaltung von Kohlendioxid ablaufende Reaktion von Isocyanatgruppen mit Wasser mit Sicherheit auszuschließen. Andererseits kann nicht vermieden werden, daß bei speziellen Anwendungsgebieten, beispielsweise bei Kabelvergußmassen, die auch bei schlechten Witterungsbedingungen unter freiem Himmel verarbeitet werden müssen, Wasser bei der Härtung zugegen ist. In diesem Fall kommt es bei üblichen PUR-Gießmassen auf Basis von Polyetherpolyolen zu einer starken Schaumbildung, selbst wenn durch Mitverwendung von Wasserabsorptionsmitteln dafür gesorgt wird, daß die unter Ausschluß von Luftfeuchtigkeit gelagerten Ausgangsmaterialien wasserfrei sind, wodurch die isolierende Wirkung des Gießharzes stark beeinträchtigt wird.

Die Schaumbildung tritt nicht auf, wenn an Stelle von Polyetherpolyolen Rizinusöl verwendet wird. Dieses Naturprodukt unterliegt jedoch in der Qualität starken Schwankungen, außerdem sind PUR-Formstoffe auf Basis dieses Polyesterpolyols bei Feuchtigkeitseinwirkung auf den ausgehärteten Formstoff sehr hydrolyseanfällig. Ein weiterer Nachteil der Masse auf Rizinusölbasis ist, daß sie bei längerer Lagerung bei höheren Temperaturen (> 50 °C) verspröden. Das schlechte Verhalten bei Wärmealterung ist auf die in der Rizinolsäure enthaltene Doppelbindung zurückzuführen. Dadurch bekommen die Gießharzmassen im Laufe der Zeit Risse, die sich nachteilig auf das elektrische Isoliervermögen auswirken.

Es ist prinzipiell bereits bekannt, Sulfonsäureester durch Reaktion von Sulfochloriden aliphatischer oder aromatischer Sulfonsäuren mit aliphatischen Alkoholen in Gegenwart von Chlorwasserstoff-Akzeptoren herzustellen (Organikum, 13. Auflage, Seite 608, VEB Deutscher Verlag der Wissenschaften). Sulfonsäureestergruppen aufweisende Polyhydroxylverbindungen auf Basis von Paraffin-sulfochloriden und mehrwertigen Alkoholen sind bislang jedoch noch nicht bekannt geworden. Völlig unvorhersehbar war demzufolge auch die ausgezeichnete Eignung dieser Polyhydroxylverbindungen in Gießharzformulierungen der obengenannten Art.

Gegenstand der Erfindung sind Sulfonsäureestergruppen aufweisende Polyhydroxylverbindungen einer mittleren Hydroxylfunktionalität von 1,5-4, erhältlich indem man

a) durch Sulfochlorierung von Paraffinen mit 10 bis 18 Kohlenstoffatomen oder von technischen Gemischen derartiger Paraffine erhaltene, gegebenenfalls im Gemisch mit nicht ungesetzten Paraffinen und/oder mit Chlorparaffinen vorliegende, Paraffinsulfochloride mit einem Gehalt an verseifbarem Chlor, bezogen auf das Gesamtgewicht der Komponente a), von 5 bis 15 Gew.-% mit

b) einem mindestens dreiwertigen aliphatischen Alkohol des Molekulargewichtsbereichs 92 bis 250 oder mit Gemischen mehrwertiger aliphatischer Alkohole des Molekulargewichtsbereichs 62 bis 250 einer mittleren Hydroxylfunktionalität von 2,5 bis 5, wobei die jeweils eingesetzten Alkohole primäre oder sekundäre Hydroxylgruppen aufweisen,

in Gegenwart von Chlorwasserstoff-Akzeptoren unter Einhaltung eines Äquivalentverhältnisses von Hydroxylgruppen zu Sulfochloridgruppen von mindestens 2 : 1 umsetzt und gegebenenfalls im Anschluß an die Umsetzung Sulfochloridgruppen-freies Paraffin bzw. Chlorparaffin und überschüssigen Alkohol aus dem Reaktionsgemisch entfernt.

Gegenstand der Erfindung ist schließlich auch die Verwendung der nach diesem Verfahren erhaltenen, Sulfonsäureestergruppen aufweisenden Polyhydroxylverbindungen, als Polyolkomponente in Gießharzformulierungen auf Basis von zu Polyurethanen ausreagierenden Gemischen aus organischen Polyhydroxylverbindungen und organischen Polyisocyanaten.

Reaktionspartner beim erfindungsgemäßen Verfahren sind

a) Paraffinsulfochloride und
b) mehrwertige Alkohole.

Geeignete Ausgangsmaterialien a) sind die an sich bekannten Sulfochlorierungsprodukte von Paraffinen mit 10 bis 18 Kohlenstoffatomen bzw. von technischen Gemischen derartiger Paraffine, wie sie in an sich bekannter Weise durch Sulfochlorierung der Paraffine, d. h. durch Behandlung mit Schwefeldioxid und Chlor oder Bestrahlung mit kurzwelligem Licht erhalten werden können. Die hierbei zum Einsatz gelangenden Paraffine können geradkettig und/oder verzweigt sein. Die beim erfindungsgemäßen Verfahren eingesetzte Komponente a) besteht im allgemeinen aus Gemischen von Paraffinsulfochloriden mit nicht ungesetzten Paraffinen und/oder mit Chlorparaffinen, wobei auch die Paraffinsulfochloride gegebenenfalls Chlorsubstituenten aufweisen können. Wesentlich für die Eignung dieser Gemische für das erfindungsgemäße Verfahren ist ihr Gehalt an verseifbarem, im wesentlichen von den Sulfochloridgruppen herrührendem Chlor. Der Gehalt des Gemische an verseifbarem Chlor liegt im allgemeinen bei 5 bis 15, vorzugsweise bei 5 bis 9 Gew.-%. Das nicht verseifbare, gegebenenfalls in Form von aliphatischen Chlorsubstituenten vorliegende Chlor geht nicht in diese Berechnung ein. Die Gemische können beim erfindungsgemäßen Verfahren als solche verwendet werden. Die Abtrennung des nicht umgesetzten Paraffins und des Sulfochloridgruppen-freien chlorierten Paraffins kann gewünschtenfalls im Anschluß an die erfindungsgemäße Umsetzung erfolgen, wie dies nachstehend noch näher ausgeführt wird.

Bei den Alkoholen b) handelt es sich um mehrwertige aliphatische Alkohole mit primären oder sekundären Hydroxylgruppen. Dabei handelt es sich entweder um mindestens dreiwertige aliphatische Alkohole des Molekulargewichtsbereichs 92 bis 250 oder um Gemische mehrwertiger aliphatischer, Alkohole des Molekulargewichtsbereichs 62 bis 250 mit einer mittleren Hydroxylfunktionalität von 2,5 bis 5. Geeignete mehrwertige aliphatische Alkohole sind beispielsweise Ethylenglykol, 1,2- oder 1,3-Dihydroxypropan, die verschiedenen isomeren Butandiole, insbesondere 1,4-Dihydroxybutan, die verschiedenen isomeren Hexandiole, insbesondere 1,6-Hexandiol, Glyzerin, Trimethylolpropan, Pentaerythrit, Sorbit oder Mannit. Ganz besonders bevorzugt wird beim erfindungsgemäßen Verfahren Trimethylolpropan verwendet.

Das erfindungsgemäße Verfahren wird in Gegenwart von Chlorwasserstoff-Akzeptoren durchgeführt. Hierzu eignen sich insbesondere tertiäre Amine wie beispielsweise Triethylamin, Tributylamin, N,N-Dimethylanilin oder Pyridin. Besonders bevorzugt wird Triethylamin verwendet, da es sich aufgrund seines niederen Siedepunkts nach beendeter Reaktion leicht aus dem Reaktionsgemisch entfernen läßt.

Bei der Durchführung des erfindungsgemäßen Verfahrens kommen die Ausgangsmaterialien a) und b) in solchen Mengen zum Einsatz, die einem Äquivalentverhältnisvon Hydroxylgruppen zu Sulfochloridgruppen von mindestens 2 : 1, vorzugsweise von 2 : 1 bis 3 : 1 entsprechen, wobei ein gegebenenfalls zum Einsatz gelangter Überschuß an Polyhydroxylverbindungen im Anschluß an die Umsetzung gewünschtenfalls entfernt werden kann. Der Gehalt der Komponente a) an Sulfochloridgruppen kann aus dem titrimetrisch ermittelbaren Gehalt an verseifbarem Chlor berechnet werden. Der Chlorwasserstoff-Akzeptor wird im allgemeinen in einer Menge von 1,02 bis 1,5 Äquivalenten, vorzugsweise von 1,1 bis 1,3 Äquivalenten, bezogen auf die Menge der in der Komponente a) vorliegenden Sulfochloridgruppen zugesetzt.

Zweckmäßigerweise wird die Umsetzung so durchgeführt, daß man die Alkoholkomponente b) und den Chlorwasserstoff-Akzeptor vorlegt, die Mischung soweit unter Rühren aufheizt, bie eine klare Lösung vorliegt und dann die Komponente a) zutropft. Hierbei kann die Heizung entfernt werden, da die Reaktion schwach exotherm verläuft. Nach beendeter Zugabe der Komponente a) läßt man das Reaktionsgemisch bei Temperaturen von 40 bis 80 °C, vorzugsweise 60 bis 70 °C nachreagieren. Die Nachreaktionszeit beträgt im allgemeinen 2 bis 10, vorzugsweise 3 bis 7 Stunden.

Nach beendeter Umsetzung wird im allgemeinen der im Überschuß vorliegende Chlorwasserstoff-Akzeptor (vorzugsweise tertiäres Amin) durch Waschen mit einer löslichen Säure (beispielsweise wäßrige Salzsäure) entfernt. In Abhängigkeit von dem Gehalt der Komponente a) an Sulfonsäureestergruppenfreien Paraffinen und/oder Chlorparaffinen bilden sich hierbei zwei oder drei Phasen aus, wobei die untere, wäßrige Phase das Hydrochlorid des Amins und den gegebenenfalls überschüssigen Alkohol und die obere(n), organische(n) Phase(n) das Verfahrensprodukt zusammen mit nicht umgesetztem Paraffin und Chlorparaffin enthalten. Nach Abtrennen der wäßrigen Phase werden die organische Phase (zweiphasig) bzw. die kombinierten organische Phase (dreiphasig) in einem geeigneten Lösungsmittel (beispielsweise Toluol) gelöst, mit verdünnter Säure (beispielsweise wäßriger Salzsäure) und anschließend mit reinem Wasser gewaschen, und anschließend durch azeotrope Destillation entwässert. Anschließend wird das Lösungsmittel destillativ abgezogen und schließlich weitere flüchtige Anteile (nicht umgesetztes Paraffin und/oder Chlorparaffin) gewünschtenfalls durch Vakuumdestillation entfernt. Eine weitgehende destillative Entfernung dieser Bestandteile gelingt beispielsweise bei 13 mbar und einer Sumpftemperatur von bis zu 180 °C, vorzugsweise von bis zu 150 °C.

Die Verfahrensprodukte fallen als Destillationsrückstand an. Es handelt sich hierbei um Sulfonsäureestergruppen aufweisende Polyhydroxylverbindungen einer mittleren Hydroxylfunktionalität von 1,5 bis 4, vorzugsweise von 1,8 bis 2,5. Die Hydroxylfunktionalität kann durch den Gehalt den Ausgangskomponente a) an Sulfonsäurechloridgruppen, die Funktionalität der Alkoholkomponente b) und durch geeignete Wahl des Äquivalentverhältnisses von Sulfonsäurechloridgruppen zu Hydroxylgruppen bei der Durchführung des erfindungsgemäßen Verfahrens eingestellt werden. Die neuen Polyhydroxylverbindungen weisen im allgemeinen eine Hydroxylzahl von 80 bis 200, vorzugsweise 100 bis 150 bei einer Säurezahl von 10 bis 40, vorzugsweise 12 bis 30 auf. Im allgemeinen handelt es sich um bei 20 °C flüssige Substanzen einer Viskosität von 400 bis 3 000 mPa · s.

Die erfindungsgemäßen Verfahrensprodukte können in Kombination mit organischen Polyisocyanaten zur Herstellung von Gießharzen verwendet werden.

Für diese erfindungsgemäße Verwendung können im Prinzip alle beliebigen, üblicherweise in der Polyurethanchemie eingesetzten Polyisocyanate verwendet werden. Beispiele hierfür sind 4,4'-Diisocyanatodiphenylmethan bzw. dessen technische Gemische mit 2,4'- und gegebenenfalls 2,2'-Diisocyanatodiphenylmethan, Polyisocyanatgemische der Diphenylmethanreihe, wie sie durch Phosgenierung von Anilin/Formaldehyd-Kondensaten in an sich bekannter Weise gewonnen werden, und die neben den genannten Diisocyanaten wechselnde Mengen an höheren Homologen enthalten, 2,4-Diisocyanatotoluol und seine technischen Gemische mit bis zu 35 Gew.-%, bezogen auf Gesamtgemisch an 2,6-Diisocyanatotoluol, Hexamethylendiisocyanat, 1-Isocyanato-3,5,5-trimethyl-5-isocyanatomethylcyclohexan (Isophorondiisocyanat), Urethan-, Carbodiimid-, Isocyanurat-, Allophanat- oder Biuret-modifizierte Polyisocyanate auf Basis der genannten unmodifizierten Polyisocyanate oder beliebige Gemische der beispielhaft genannten Polyisocyanate. Vorzugsweise werden die entsprechenden, bei Raumtemperatur flüssigen Polyisocyanate verwendet. Besonders bevorzugt werden als organische Polyisocyanate die Phosgenierungsprodukte von Anilin/Formaldehyd-Kondensaten verwendet.

Zur Herstellung der Gießharze werden die erfindungsgemäßen, Sulfonsäureestergruppen aufweisenden Polyhydroxylverbindungen, gegebenenfalls in Kombination mit bis zu 50 Hydroxyläquivalent-% an anderen Polyhydroxylverbindungen mit den Polyisocyanaten vermischt. Hierbei liegt das Äquivalentverhältnis von Isocyanatgruppen zu Hydroxylgruppen bei 0,75 : 1 bis 1,25 : 1, vorzugsweise bei 0,9 : 1 bis 1,1 : 1.

Bei den gegebenenfalls neben den erfindungsgemäßen Polyhydroxylverbindungen mitzuverwendenden weiteren Polyhydroxylverbindungen handelt es sich in erster Linie um Polyetherpolyole einer (mittleren) Hydroxylfunktionalität von 2 bis 4 und eines (mittleren), aus dem Hydroxylgehalt und der Hydroxylfunktionalität berechenbaren Molekulargewichts von 400 bis 1 200. Gut geeignet sind insbesondere die Propoxylierungsprodukte von mehrwertigen Alkoholen der bei der Beschreibung der Komponente b) oben genannten Art, wobei selbstverständlich das Startermolekül bei der Herstellung der Polyetherpolyole mit dem beim erfindungsgemäßen Verfahren eingesetzten Alkohol b) nicht identisch sein muß. Grundsätzlich ist es auch möglich, neben derartigen Polyetherpolyolen andere Polyhydroxylverbindungen, beispielsweise niedermolekulare Polyhydroxylverbindungen der bei der Beschreibung der Komponente b) genannten Art und/oder Rizinusöl bei der Formulierung der Gießharze mit zu verwenden, was allerdings weniger bevorzugt ist. Auf jeden Fall besteht die Polyolkomponente in den erfindungsgemäßen Gießharzen zu mindestens 50 Hydroxyläquivalent-% aus den erfindungsgemäßen, Sulfonsäureestergruppen aufweisenden Polyhydroxylverbindungen.

Den Gießharzen können gewünschtenfalls übliche Zusatzmittel zugesetzt werden. Hierzu gehören beispielsweise Zeolith (wasserfrei) in einer Menge von bis zu 5 Gew.-%, bezogen auf Gesamtgemisch, Füllstoffe wie gemahlenes Gesteinsmahl, Kurzglasfasern, Polyethylenpulver oder sonstige anorganische oder organische Füllstoffe, Pigmente oder aus der Polyurethanchemie an sich bekannte Beschleuniger.

Die Gießharze weisen im allgemeinen bei Raumtemperatur eine Topfzeit von 0,25 bis 10 Stunden auf und härten bei Raumtemperatur innerhalb eines Zeitraums von 2 bis 24 Stunden zu blasenfreien Kunststoffen aus, wobei die Topf- und Aushärtezeit insbesondere durch die An- bzw. Abwesenheit von Beschleunigern für die Isocyanat-Polyadditionsreaktion bestimmt wird. Gewünschtenfalls kann diese Zeit durch Temperaturerhöhung wesentlich verkürzt werden.

Die erfindungsgemäßen Gießharze eignen sich insbesondere als Vergußmassen in der Elektroindustrie zur Herstellung von Isolatoren.

In den nachfolgenden Beispielen beziehen sich alle Prozentangaben auf Gewichtsprozente.


Beispiel 1


In einem Rundkolben, versehen mit Rührer, Rückflußkühler und Thermometer werden 670 g Trimethylolpropan (5 mol) und 602 g Triethylamin (6 mol) vermischt und unter Rühren auf 60 °C aufgeheizt. Anschließend werden 2 650 g eines « Paraffinsulfochlorids » mit einem Gehalt an verseifbarem Chlor von 7 % innerhalb von 2 Stunden zugetropft. Das zum Einsatz gelangende « Paraffinsulfochlorid » wurde durch Sulfochlorierung eines technischen Paraffingemischs mit einer mittleren Kettenlänge von 11 Kohlenstoffatomen erhalten. Es handelt sich um ein Gemisch, welches zu ca. 50 Gew.-% aus Paraffin-sulfochlorid und zum Rest aus nicht umgesetzten Paraffinen und Chlorparaffinen besteht. Das Äquivalentverhältnis von Hydroxylgruppen zu Sulfochloridgruppen liegt bei ca. 3 : 1. Während des Zutropfens des « Paraffinsulfochlorids » wird eine Reaktionstemperatur von 60-70 °C aufrechterhalten. Nach beendeter Zugabe rührt man noch 4 Stunden bei 60-70 °C nach. Anschließend wird das Reaktionsgemisch zweimal mit jeweils 250 ml 10 %iger Salzsäure gewaschen. Hierbei bilden sich drei Phasen, von denen die untere, wäßrige Phase das Hydrochlorid des Amins enthält. Die mittlere Phase enthält die Hauptmenge des Verfahrensproduktes im Gemisch mit untergeordneten Mengen an Paraffinen und Chlorparaffinen, während die obere Phase im wesentlichen aus nicht umgesetzten Paraffinen und Chlorparaffinen besteht. Die beiden oberen Phasen werden kombiniert, in 500 ml Toluol gelöst und mit 250 ml 10 %iger Salzsäure und anschließend mit 250 ml Wasser gewaschen. Anschließend wird die Toluollösung azeotrop entwässert und das Toluol bis zu einer Sumpftemperatur von 140 °C destillativ entfernt. Nach Abkühlen auf 60 °C werden flüchtige Anteile im Vakuum (13 mbar) destillativ abgezogen, wobei die Sumpftemperatur in der Endphase der Destillation bis auf 150 °C ansteigt.

Als Destillationsrückstand fallen so 2 023 g einer erfindungsgemäßen, Sulfonsäureestergruppen aufweisenden Polyhydroxylverbindung der Säurezahl 13, der Hydroxylzahl 147 und der Viskosität (20 °C) 2 200 mPa · s an.

Die Hydroxylzahl errechnet sich aus Hydroxylfunktionalität × 56 100 dividiert durch das Molekulargewicht.

## Beispiel 2

100 g der erfindungsgemäßen Polyhydroxylverbindung aus Beispiel 1 werden mit 10 g eines Gemischs aus 50 % Rizinusöl und 50 % wasserfreiem Zeolith gemischt. Anschließend werden 37 g eines Polyisocyanatgemischs der Diphenylmethanreihe (Phosgenierungsprodukt eines Anilin/Formaldehyd-Kondensats mit einem NCO-Gehalt von 31 % und einer Viskosität bei 23 °C von 120 mPa · s) eingerührt. Die so erhaltene Gießharzmasse weist eine Topfzeit von ca. 8,5 Stunden auf. An einer 4 mm starken Platte, die 24 Stunden bei Raumtemperatur und 24 Stunden bei 80 °C gehärtet worden war, konnten folgende Eigenschaften gemessen werden :

| | |
|---|---|
| Shore Härte A : | 35 |
| Einreißfestigkeit : | 0,85 N/m$^2$ |
| Dehnung : | 55 % |
| Wasseraufnahme nach 24 h | |
| Lagerung bei R.T. : | 40 mg. |

## Beispiel 3

80 g der erfindungsgemäßen Polyhydroxylverbindung aus Beispiel 1 werden mit 20 g eines Hydroxylgruppen aufweisenden Polyetherpolyols mit einem Hydroxylgruppengehalt von 11 % und einer Viskosität bei 25 °C von 600 mPa · s (Propoxylierungsprodukt von Trimethylolpropan) und 10 g Zeolithpaste gemäß Beispiel 2 gemischt. Anschließend wird diese Polyolkomponente mit 44 g des Polyisocyanat gemäß Beispiel 2 vermischt. Die resultierende Gießharzformulierung weist bei Raumtemperatur eine Topfzeit von 100 min auf. Nach der Härtung während 24 h bei Raumtemperatur und anschließend während 24 h bei 80 °C können an einer 4 mm dicken Platte folgende Werte gemessen werden :

| | |
|---|---|
| Shore Härte A : | 99 |
| Shore Härte D : | 67 |
| Einreißfestigkeit : | 17,1 N/mm$^2$ |
| Dehnung : | 2,5 % |
| Kerbschlagzähigkeit : | 2,6 kJ/m$^2$ |
| Wasseraufnahme : | 23,9 mg. |

## Beispiel 4

50 g der in Beispiel 3 beschriebenen Polyurethangießmasse werden in ein Becherglas gegossen, in dem sich 5 g Wasser befinden. Die Aushärtung der Gießharzmasse erfolgt bei Raumtemperatur in Gegenwart von Wasser. Nach der Härtung erhält man einen kompakten Gießling, der auf der Oberfläche keinerlei Schaumbildung zeigt.

## Beispiel 4a (Vergleichsbeispiel zu Beispiel 4)

Beispiel 4 wird wiederholt mit dem einzigen Unterschied, daß in der Formulierung die erfindungsgemäße Polyhydroxylverbindung gemäß Beispiel 1 durch eine äquivalente Menge eines Polypropylenglykols der OH-Zahl 90 ersetzt wird. Bei der Aushärtung in Gegenwart von Wasser schäumt das Reaktionsgemisch auf das etwa doppelte Volumen auf.

## Patentansprüche

1. Sulfonsäureestergruppen aufweisende Polyhydroxylverbindungen einer mittleren Hydroxylfunktionalität von 1,5 bis 4, erhältlich indem man

a) durch Sulfochlorierung von Paraffinen mit 10 bis 18 Kohlenstoffatomen oder von technischen Gemischen derartiger Paraffine erhaltene, gegebenenfalls im Gemisch mit nicht ungesetzten Paraffinen und/oder mit Chlorparaffinen vorliegende, Paraffinsulfochloride mit einem Gehalt an verseifbarem Chlor, bezogen auf das Gesamtgewicht der Komponente a), von 5 bis 15 Gew.-%

mit

b) einem mindestens dreiwertigen aliphatischen Alkohol des Molekulargewichtsbereichs 92 bis 250 oder mit Gemischen mehrwertiger aliphatischer Alkohole des Molekulargewichtsbereichs 62 bis 250 mit einer mittleren Hydroxylfunktionalität von 2,5 bis 5, wobei die jeweils eingesetzten Alkohole primäre oder sekundäre Hydroxylgruppen aufweisen, in Gegenwart von Chlorwasserstoff-Akzeptoren unter Einhaltung eines Äquivalentverhältnisses von Hydroxylgruppen zu Sulfochloridgruppen von mindestens 2 : 1 umsetzt und gegebenenfalls im Anschluß an die Umsetzung Sulfochloridgruppen-freies Paraffin bzw. Chlorparaffin und überschüssigen Alkohol aus dem Reaktionsgemisch entfernt.

2. Sulfonsäureestergruppen aufweisende Polyhydroxylverbindungen gemäß Anspruch 1, dadurch gekennzeichnet, daß man als Komponente b) Trimethylolpropan verwendet.

3. Verwendung von Sulfonsäureestergruppen aufweisenden Polyhydroxylverbindungen gemäß wenigstens einem der vorhergehenden Ansprüche als Polyhydroxylkomponente in Gießharzformulierungen auf Basis von zu Polyurethanen ausreagierenden Gemischen aus organischen Polyhydroxylverbindungen und organischen Polyisocyanaten.

## Claims

1. Polyhydroxyl compounds containing sulfonic acid ester groups and having an average hydroxyl functionality of 1.5 to 4, obtainable by reaction of

a) paraffin sulfochlorides, optionally present in admixture with unreacted paraffins and/or with chloroparaffins, obtained by sulfochlorination of paraffins containing 10 to 18 carbon atoms or of technical mixtures of such paraffins and containing 5 to 15 % by weight hydrolyzable chlorine, based on the total weight of component a), with

b) an at least trihydric aliphatic alcohol having a molecular weight of 92 to 250 or with mixtures of polyhydric aliphatic alcohols having a molecular weight of 62 to 250 and an average hydroxyl functionality of 2.5 to 5, the particular alcohols used containing primary or secondary hydroxyl groups, in the presence of hydrogen chloride acceptors, an equivalent ratio of hydroxyl groups to sulfochloride groups of at least 2 : 1 being maintained during the reaction, and — optionally — removal of paraffin or chloroparaffin free from sulfochloride groups and excess alcohol from the reaction mixture after the reaction.

2. Polyhydroxyl compounds containing sulfonic acid ester groups as claimed in claim 1, characterized in that trimethylol propane is used as component b).

3. The use of the polyhydroxyl compounds containing sulfonic acid ester groups claimed in at least one of the preceding claims as polyhydroxyl component in casting resin formulations based on reactive polyurethane-forming mixtures of organic polyhydroxyl compounds and organic polyisocyanates.

## Revendications

1. Composés polyhydroxylés, présentant des groupes esters d'acide sulfonique et ayant une fonctionnalité hydroxyle moyenne (un nombre moyen de groupes hydroxyles fonctionnels) de 1,5 à 4, que l'on peut obtenir en faisant réagir, en présence d'accepteurs de chlorure d'hydrogène, en maintenant un rapport entre les équivalents des groupes hydroxyles et ceux des groupes sulfochlorures d'au moins 2 : 1,

a) des sulfochlorures de paraffines (obtenus par sulfochloruration de paraffines ayant 10 à 18 atomes de carbone ou de mélanges techniques de telles paraffines, éventuellement présents en mélange avec des paraffines n'ayant pas réagi et/ou avec des chloroparaffines), ayant une teneur en chlore saponifiable, sur la base du poids total du composant a), de 5 à 15 % en poids, avec

b) un alcool aliphatique au moins trivalent (un polyalcool qui est au moins un trialcool) dont le poids moléculaire se situe entre 92 et 250, ou avec des mélanges de polyalcools aliphatiques dont le poids moléculaire se situe entre 62 et 250 et ayant une fonctionnalité hydroxyle moyenne (un nombre moyen de groupes hydroxyles) de 2,5 à 5, les alcools utilisés à chaque fois présentant des groupes hydroxyles primaires ou secondaires. .

2. Composés polyhydroxylés, présentant des groupes esters d'acide sulfonique, selon la revendication 1, caractérisé en ce qu'on utilise, comme composant b), le triméthylolpropane.

3. Utilisation de composés polyhydroxylés, présentant des groupes esters d'acide sulfonique, selon au moins une des revendications précédentes, comme composant polyhydroxylé dans des formulations de résines pour coulée, à base de mélanges de composés polyhydroxylés organiques et de polyisocyanates organiques à faire réagir pour obtenir des polyuréthannes.